(19) Europäisches Patentamt

European Patent Office

Office européen des brevets

(11) Publication number: **0 357 208**

**A1**

(12) **EUROPEAN PATENT APPLICATION**

(21) Application number: 89307406.2

(22) Date of filing: 20.07.89

(51) Int. Cl.5: **A61K 39/112 , C12N 15/00 , A61K 39/015 , //(C12N15/00, C12R1:42),(C12N15/00, C12R1:90)**

Claims for the following Contracting States: GR + ES.

(30) Priority: 21.07.88 US 222202

(43) Date of publication of application:
07.03.90 Bulletin 90/10

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

Applicant: UNITED STATES OF AMERICA as represented by THE SECRETARY OF THE ARMY
Department of Army U.S. Army Legal Services Agency Nassif Building
Falls Church, VA 22041-5013(US)

(72) Inventor: Sadoff, Jerald Charles
1622 Kalmia Road
Washington, DC 20012(US)
Inventor: Young, James Francis
12624 Gravenhurst Lane
Gaithersburg MD 20878(US)

(74) Representative: Giddings, Peter John, Dr. et al
Smith Kline & French Laboratories Ltd.
Corporate Patents Mundells
Welwyn Garden City Hertfordshire AL7 1EY(GB)

(54) Salmonella transformant capable of expression of heterologous genes and useful as a recombinant vaccine.

(57) The invention provides a Salmonella baceterial strain transformed with an expression vector comprising a heterologous gene operatively linked to an E. coli promoter sequence, the transformed strain being capable of constitutive expression of the product of the heterologous gene. Also provided is an attenuated Salmonella strain containing all or a portion of a selected circumsporozoite protein, useful for inducing protective cell-mediated immunity to malarial parasites.

# SALMONELLA TRANSFORMANT CAPABLE OF EXPRESSION OF HETEROLOGOUS GENES AND USEFUL AS A RECOMBINANT VACCINE

The present invention relates generally to a Salmonella bacterial transformant containing a heterologous gene capable of inducing protective immunity against an infective agent, the gene being under the regulatory control of an E. coli promoter. More specifically, the invention provides a Salmonella transformant capable of constitutive expression of a Plasmodium circumsporozoite gene, for use as a vaccine capable of inducing protective cell-mediated immunity against malaria in mammals.

## BACKGROUND OF THE INVENTION

Salmonella is a bacterial species which, upon infection, causes gastrointestinal disease in mammals. For immunization against such infection, various strains of Salmonella have been mutagenized for use as live vaccines or have been attenuated to reduce the virulence of the strain by a variety of mutagenic techniques. United States Patent 4,550,081 discloses mutagenized live Salmonella vaccines. Attenuated strains, such as the aroA mutant, are described in S. K. Hoiseth et al, Nature, 291: 233-9 (1981).

Attenuated Salmonella strains are known to rapidly translocate from the site of inoculation by way of regional and systemic lymphatics and are ingested by macrophages within the reticuloendothelial system. [A.A. Lindberg et al, Infect Immun., 41:751 (1983)]. Protective immunity induced by attenuated Salmonella against infection by that bacteria has been determined to be cell-mediated. Its induction requires a relatively long period of intracellular survival by the organism. [See, e.g. T.K. Eisenstein, J. Immunol., 133:958(1984); L.M. Killer et al, J. Immunol; 133:1190 (1984); and A. Tagliabue et al, Clin Exp. Immunol., 62:242 (1985)]

Salmonella strains have also recently been described as transformants for the development of a vaccine to immunize against Streptococcus mutans-induced dental caries. R. Curtiss III, et al., in "Vaccines:new concepts and developments", eds. H. Kohler et al, Longman Scientific and Technical, NY, NY (1988) pp. 261-271 describe a Salmonella strain transformed with a Strep. mutans bacterial gene under the control of the S. mutans promoter and administered orally to encourage an immunogenic response directed primarily in the oral cavity.

Years of extensive effort have also been devoted to the development of an effective vaccine against malaria, a severe and widespread disease, caused by various species of the protozoan parasite genus Plasmodium. See, e.g., Science,226:679 (1984). Four species of Plasmodium infect man, e.g., P. falciparum, P. vivax, P. ovale and P. malariae and others, e.g., P. berghei and P. knowlesi, infect rodents and monkeys, respectively. The focus of much of this effort is the sporozoite form of the Plasmodium parasite which is accessible in the bloodstream after a mammal is bitten by a female Anopheline mosquito.

Experimentally, mammals, including man, have been protected against infection by vaccination with whole irradiated sporozoites. The immunogenicity of sporozoites has been determined to reside largely in the circumsporozoite (CS) protein, which covers the surface of the sporozoite. [Clyde et al., Am. J. Trop. Med. Hyg., 24:397 (1975) and Rieckman et al., Bull, WHO, 57 (Supp. 1):261 (1979).] Yoshida et al., Science, 207:71 (1980) report that the protection resulting from immunization with irradiated sporozoites is at least partially mediated by antibody directed against the CS protein. Monoclonal antibodies raised against CS proteins neutralize infectivity in vitro and protect animals in vivo.

Recent research has resulted in the cloning and characterization of the genes or subunits thereof for the CS proteins of a number of the plasmodia species. Expression of these cloned genes or portions thereof has generally been in E. coli or yeast vectors and host cells. The first successful cloning of a CS protein was from the simian malarial parasite, P. knowlesi. Zavala et al., J. Exp. Med., 157:1947 (1983), report that a twelve amino acid sequence repeated tandomly twelve times in the genome is the major immunogen on the P. knowlesi CS protein. Godson et al, Nature, 305:29-33 (1983) disclose the cDNA of an epitope of P. knowlesi CS protein, which competes in competition assays with whole sporozoite antigen for monoclonal antibodies directed against the sporozoite. Gysin et al, J. Exp. Med., 160:935 (1984), report that a synthetic 24 residue peptide representing tandem repeat units of the P. knowlesi CS protein neutralized infectivity of virulent sporozoites in monkeys.

Colman et al., WO 84-2922-A, published August 2, 1984, cloned a portion of the coding region for the P. knowlesi CS protein repeat unit and expressed it as a fusion protein with beta-lactamase and beta-galactosidase sequences in E. coli.

Sharma et al, Science, 228:879-882 (1985) purified the P. knowlesi CS protein precursor from expression in the yeast Saccharomyces cerevisiae.

EP 0 357 208 A1

Dame et al., Science, 225:593 (1984), report cloning and expression of the CS protein of P. falciparum in E. coli and the immunogenicity of synthetic 7-, 11-and 15- residue peptides derived therefrom.

Vergara et al, PCT application WO86/01721, published March 27, 1986, disclose a peptide from a non-repetitive epitope of the P. knowlesi CS protein, capable of eliciting antibodies to the CS protein and thus useful as a vaccine candidate.

Nussenzweig et al., U.S. 4,466,917, disclose a sporozoite protein referred to as the P44 protein and its cloning and expression in E. coli. Kemp et al., WO.84-02917-A, disclose cloning and expression of P. falciparum cDNA in E. coli.

Enea et al., Proc. Natl. Acad. Sci. USA, 81:7520 (1984), report an analogous repeat unit structure within the CS protein of P. cynomolgi. Ellis et al, PCT application WO86/00911, published February 13, 1986, disclose the use of a peptide repeat forming the epitope of the P. falciparum CS protein in a subunit vaccine for malaria.

McCutchan et al, U.S. Patent 4,693,994, issued September 15, 1987, disclose a synthetic peptide based on the CS protein of P. vivax, which is capable of inducing antibodies protective against malarial infection.

To date, vaccines based on the use of the CS surface antigen or subunits thereof from various Plasmodium species have primarily elicited humoral immune responses of the host against the repeat region of the CS protein. There remains a need in the field for additional vaccines efficacious against these parasites and other infectious organisms.


SUMMARY OF THE INVENTION

The present invention thus provides a Salmonella bacterial strain transformed with a selected heterologous gene operatively linked to an E. coli promoter sequence, the transformant capable of constitutively expressing the product of the heterologous gene. The selected heterologous gene preferably encodes a protein or portion thereof from an infective agent, e.g., a virus, bacteria, or parasite, which is capable of eliciting cell-mediated immune protection in an animal or human directed against the infective agent from which the heterologous gene originates.

Another aspect of this invention is an attenuated Salmonella strain transformed with a plasmid containing a selected heterologous gene from an infective agent under the control of the bacteriophage lambda leftward promoter $P_L$. Such Salmonella transformants are capable of constitutive expression of the heterologous gene in the absence of a cl repressor protein. The cl repressor protein is ordinarily required to regulate the function of the strong $P_L$ promoter in E. coli cells.

As a further aspect of this invention a novel vaccine against malaria is disclosed, which is capable of eliciting an antigen-specific cell-mediated immune response against Plasmodium sporozoites in the host. This vaccine is composed of an attenuated Salmonella bacterial transformant according to the invention containing a plasmid having a gene encoding a Plasmodium CS protein of interest or a subunit thereof under the control of an E. coli promoter. In a preferred embodiment, the gene encodes substantially the full length CS protein as Salmonella has now been found to be capable of expressing a full length Plasmodium CS protein. Also, the full length CS protein has now been found to elicit a more protective response than a partial CS protein.

Also encompassed by this invention are other vaccines prepared from the transformed Salmonella strains of the invention, particularly vaccines comprising an attenuated Salmonella strain tranformed with a plasmid containing a gene encoding a protein of another infective agent, e.g., a virus such as HIV-1, HIV-2 or HIV-3 under the control of an E. coli promoter. These vaccines are of particular interest for use against any infectious disease for which cell-mediated immunity is an important component of protection, e.g. leprosy, leishmaniasis, schistomiasis, rickettsia and AIDS.

Still a further aspect of the present invention is a method for expressing a selected heterologous gene in a Salmonella bacterial strain by culturing in a suitable medium a Salmonella transformant of the invention. The gene product so expressed may be a protein isolated by conventional means and itself used in a vaccine or other composition, or any desirable heterologous gene product, e.g. tissue plasminogen activator, interferon, hepatitus B surface antigen or a gene product useful as a diagnostic reagent.

This invention also provides a method for inducing cell-mediated immunity against infection by an infective agent comprising administering to a subject an effective amount of an attenuated Salmonella bacterial transformant according to the invention. The transformant contains a vector in which the coding sequence of all, or a portion of, a gene from a selected infective agent capable of inducing protective immunity, is under the control of an E. coli promoter. The transformant is capable of constitutive expression of the heterologous gene in vivo.

3

The invention also provides a method for vaccinating an animal or human against infection by an infective agent by co-administering a vaccine of the invention with another vaccinal agent capable of inducing an antibody response to the infective agent.

Other aspects and advantages of the present invention are disclosed in the following detailed description.

BRIEF DESCRIPTION OF THE DRAWINGS

Fig. 1 is a partial restriction endonuclease cleavage map of a region of the P. berghei CS protein coding region.

Fig. 2 illustrates the construction of the plasmid pMGB2 containing the entire P.berghei CS protein coding region, the lambda $P_L$ promoter, an origin of replication and a gene for ampicillin resistance.

DETAILED DESCRIPTION OF THE INVENTION

The present invention provides a novel source of expression of heterologous genes, as well as a novel vaccine component capable of constitutively expressing a gene from an infectious organism which may induce an in vivo immunogenic response in a host to the invading organism. This invention provides in its broadest aspect a Salmonella bacterial strain transformed with an expression vector containing a heterologous gene operatively linked to an E. coli promoter sequence.

Where the transformants of this invention are used to express a selected heterologous gene in vitro, any normal strain of Salmonella may be employed, e.g. S. dublin, S. typhii, S. typhimurium. However, presently preferred for in vivo use and for convenient laboratory bench use are attenuated strains of Salmonella. Many such strains are widely available, e.g. S. typhimurium strain WR4017 (available from the Walter Reed Culture Collection) and S. typhii strains galE (Ty21A) (available from the Swiss Serum Institute, Switzerland) and aroA (541 Ty) (available from the American Type Culture Collection, Rockville, Md.). Appropriate attenuated Salmonella strains for use in this invention may be obtained from the American Type Culture Collection, other commercial collections, or scientific laboratories. Alternatively, a selected Salmonella strain may be attenuated by conventional·methods, such as described in Lindberg, supra, incorporated herein by reference. The transformants of the invention are not limited to any particular strains.

The invention is similarly unlimited as to the particular E. coli promoter employed to control the expression of the heterologous gene in the Salmonella strain. Preferred promoters are the strong E. coli promoters, such as the major leftward and rightward promoters of bacteriophage lambda. Other known E. coli promoters, such as trp, lac, tac, rac, and the like may also be employed in the novel transformants.

The strong $P_L$ promoter is presently preferred due to its unexpected success in the transformants of the invention. Because the $P_L$ promoter of lambda overexpresses the products of the genes whose expression it controls, a cell into which the $P_L$ promoter is transformed for expression of a gene normally requires the presence of the cl repressor protein for regulation of the promoter. In most instances the cl protein is introduced into a host by a lysogenic lambda bacteriophage. The Salmonella transformants of the present invention containing the $P_L$ promoter controlling expression of a heterologous gene provided good expression levels without the presence of the cl repressor protein, which does not normally exist in Salmonella strains. This level of expression was unexpected where the $P_L$ promoter was unregulated by the cl protein in the Salmonella host, because ordinarily the unregulated $P_L$ promoter will "exhaust" the host cell by over-expression of a product under its control. $P_L$ directed expression of the heterologous gene in Salmonella did not exhaust the capacity of the host to express the gene product according to this invention. Thus illustrative transformed Salmonella strains of the invention are characterized by the ability to express heterologous genes under the control of the $P_L$ promoter constitutively in vitro or in vivo without the presence of the cl repressor. Alternatively, the attenuated Salmonella strains of this invention could be modified to contain either the wild-type or temprature sensitive cl protein by conventional methods, e.g., $P_L$ transduction. See, e.g., US Patent 4,637,980.

The transformant of the invention may be designed to express any heterologous gene, and the invention is thus unlimited as to the heterologous gene selected. However, the transformants of the invention are presently most useful in the expression of a heterologous gene from an infectious microorganism, which gene is capable of eliciting an immunogenic response against the infective agent. For example, a transformant according to this invention may be developed to constitutively express an immunogenic protein from a bacteria, virus or parasite under control of an E. coli promoter. While the examples below illustrate the use of a CS protein, or portion thereof, of P. berghei as the heterologous gene, any gene may be employed in its place, e.g., an HIV-1 protein.

To construct a Salmonella transformant of the present invention, an attenuated strain is transformed by conventional techniques by a vector containing all or a selected portion of a heterologous gene, e.g. a portion of a gene from an infectious agent capable of inducing protective immunity, such as a CS subunit or whole gene from a desired Plasmodium species. DNA transformation, transduction or conjugation techniques between the Salmonella and another strain carrying an appropriate vector are well known in the art. Conventional techniques for Salmonella transformation are described in J.Ou et al, "Rec. Advances in Chemotherapy", J. Ishigami (ed.), Univ. of Tokyo Press, Tokyo (1985), p 383; S. Cohen et al, Proc. Natl. Acad. Sci., U.S.A., 69: 2110 (1972); E. N. Lederberg et al, J. Bacteriol., 119: 1072 (1974); and J. C. Sadoff et al, Science, 240: 336 (1988). Gene sequences of most of the Plasmodium CS antigens or subunits thereof are known and available to those of skill in the art, as are immunogenic genes from other infectious agents, e.g., viruses, bacteria. Additionally the subunit sequences of CS genes, identified recently and in the art may also be used in a vector of the present invention for transformation into the Salmonella bacterium. Other malarial stage-specific antigens may also be the product of expression of the heterologous gene, even antigens expressed by intracellular stages of the parasite, e.g. asexual stages. Coding sequences for the desired CS or other proteins or subunits thereof may be obtained by known techniques, including synthesis, reverse transcription of mRNA, or by directly cloning the intact gene from genomic DNA.

A vector for use in transformation of an attenuated Salmonella strain according to the invention may contain a number of conventional elements therein, provided that it contains an E. coli promoter to direct the expression of the heterologous gene, is capable of replicating in Salmonella and has an Enterobacteriae origin of replication. The origin of replication for use in this vector may be from any member of the Enterobacteriae family, e.g. Salmonella, E. coli. In the examples below, an E. coli ori is employed. Other known sequences, e.g. selection markers, banks of restriction enzyme sites, and the like may be optionally included in a vector for transformation into a Salmonella bacterial strain.

One illustrative vector is desirably derived from the pAS1 expression vector described in United States Patent 4,578,355, which is incorporated by reference herein, and Rosenberg et al, Meth. Enzymol., 101:123 (1983) and Shatzman et al, "Experimental Manipulation of Gene Expression, eds, M. Inouye, Academic Press, New York (1982). pAS1 carries the pBR322 origin of replication, an ampicillin resistance marker and a series of fragments from lambda, including $P_L$, N anti-termination function recognition sites (NutL and NutR), the rho-dependent transcription termination signal (tR1) and the cII ribosome binding site, including the cII translation initiation codon, the G residue of which is followed by a Bam HI cleavage site. The coding sequence of the selected CS gene is expressed well using these regulatory elements, particularly the $P_L$ promoter of lambda and the cII ribosome binding site of lambda. pAS1 is available from the American Type Culture Collection, Rockville, Maryland as described in the '355 patent.

A heterologous gene for use in the invention, e.g. the coding sequence of a selected CS gene, is operatively linked, i.e., in correct orientation and in proper reading frame, to a regulatory element of the expression vector as described in U.S. Patent 4,578,355 such as by direct fusion to the cII translational regulatory signal of pAS1, by standard techniques to construct an expression vector of the present invention.

Other known vectors and vector components may also be manipulated for use in developing a Salmonella transformant of this invention by methods analogous to those described above and in the following examples.

In addition to transformation with stably maintained plasmids, an expression unit comprising a regulatory function, e.g., a promoter, operatively linked to a coding sequence for the protein of interest can be integrated into the Salmonella chromosome by, e.g., recombination such as by use of homologous flanking regions or by use of transposons [see, e.g., U.S. Patent 4,637,980; Ruvkin, Nature 289:85 (1981) and European patent application EP-A-125,228, all being incorporated by reference herein] or by transduction such as with recombinant P22 phage. Homologous recombination is preferred because the site of integration can be controlled and because integration can be effected without need for presence of antibiotic selection markers or other selection markers in the final host. An illustrative process is as follows. First, an attenuated Salmonella strain is transformed with a plasmid comprising a tetracycline resistance marker flanked by Salmonella chromosomal DNA sequences. The cells are grown in an absence of tetracycline in order that non-integrating tetracycline genes will be lost due to plasmid instability. Then, tetracycline resistant transformants, i.e., integrants, are transformed with a second plasmid having the expression unit of the invention flanked by the same Salmonella chromosomal sequences as a result of which in some integrants, the tetracycline resistance marker will be replaced with the gene expression unit of interest. To select these cells, the cells are grown in the presence of both ampicillin and tetracycline. The cells retaining the tetracycline resistance gene will be killed by the ampicillin. The cells in which the tetracycline resistance gene has been replaced by the expression unit of interest will remain in stationary phase and therefore will not be killed by the ampicillin. Upon removal of both antibiotics, the cells having the expression unit of interest integrated therein will return to normal growth.

The attenuated, transformed Salmonella strains of the invention are also capable, upon oral administration as a vaccine, of constitutively producing the product of the heterologous gene in vivo. The transformants are capable of colonizing the liver, where the expression of the heterologous protein by the attenuated strain can induce antigen-specific cell-mediated immunity, protective against challenge by the infectious agent from which the heterologous gene originates.

A specific example disclosed below is a vaccine capable of eliciting cell-mediated immunity against malarial infection in the absence of anti-sporozoite antibodies, which consists of an attenuated Salmonella bacterial strain transformed with a vector containing a coding sequence for all, or a portion of, a circumsporozoite protein of a selected Plasmodium species under control of a bacteriophage lambda $P_L$ promoter sequence. This transformant according to the invention is capable of constitutive expression of the CS gene in the absence of a cI repressor protein. The examples below demonstrate this constitutive expression in vivo of the CS protein of P. berghei.

Unlike malaria vaccines of the prior art, the present invention surprisingly provides a vaccine based on expression of the CS protein or a subunit thereof, capable of inducing cell-mediated immunity against the sporozoite form of the parasite in the absence of significant induction of anti-sporozoite antibodies. An example of the ability of a transformant of the present invention to induce such immunity is also demonstrated in the examples below.

While the examples illustrate a vaccine of the invention employing the CS gene of P. berghei, other CS genes or subunits, most desirably the CS genes, or fragments thereof, from the human infective parasites, e.g. P. falciparum, P. malariae, P. vivax and P. ovale, may be employed in vectors for use in these vaccines to provide cell-mediated immunity against these parasitic infections. Cloning of the P. falciparum CS protein gene, e.g., is disclosed by Dame et al, Science 225:593 (1988), incorporated herein by reference. See, also, McCutchan et al, U. S. Patent 4,707,445, incorporated herein by reference. Other genes of heterologous infectious agents may be employed to construct vaccines according to the invention.

Also included in the invention is a method for expressing selected heterologous genes, such as the malarial CS antigens, in vitro by culturing in a suitable medium a Salmonella transformant containing the heterologous gene under the control of an E. coli promoter sequence. This method is most preferably performed using a transformant containing the $P_L$ promoter, whereby the transformant constitutively produces the product of the gene. After transformation, the heterologous gene product may be expressed by growing the Salmonella in culture. Suitable culture conditions and media for growth of Salmonella strains include those described in Hoiseth et al, supra and Sadoff et al, supra and references cited therein. Selection of media and conditions for in vitro growth of Salmonella is within the knowledge of one of skill in the art.

The invention also encompasses a method for inducing cell-mediated immunity against infection by an infective agent by administering to a subject an effective amount of an attenuated Salmonella bacterial strain transformed with a vector containing the coding sequence of all, or a portion of, a gene from the infective agent capable of inducing protective immunity, the gene being under control of an E. coli promoter sequence.

The transformant of the invention is capable of constitutive expression of the heterologous gene in vivo. The bacterial transformant of the examples constitutively expresses the CS antigen, and should produce constitutive expression of any other heterologous immunogenic gene from another infectious agent, upon its colonization in the liver of the host. The Salmonella transformant survives for a relatively long period intracellularly, and enables induction of cell-mediated immunity.

The vaccine of the present invention also has been shown to be effective in providing protection against an infective agent when co-administered with a vaccinal agent capable of inducing an antibody response to the infective agent. An example of such co-administration is described below in Example 5.

The mode of administration of the vaccine of the invention may be any suitable route which delivers the vaccine to the host. However, the vaccine is preferably administered by the oral route, which delivers the Salmonella strains directly into the gut. Other modes of administration may also be employed, where desired, such as parenteral administeration, e.g. subcutaneously, intradermally, intranasally, intramuscularly or intravenously.

Vaccines of the invention may be prepared as pharmaceutical compositions containing an effective amount of the attenuated Salmonella as an active ingredient in a nontoxic and sterile pharmaceutically acceptable carrier. In the vaccine of the invention, an aqueous suspension or solution containing the attenuated bacterial strain, preferably buffered at physiological pH, in a form ready for oral ingestion is

6

preferred. Alternatively where the administration of the vaccine is parenteral, the bacteria can be admixed or adsorbed with any of a conventional adjuvant, for use as non-specific irritants to attract or enhance an immune response. Such adjuvants include, among others, aluminum hydroxide, muramyl dipeptide and saponins, such as Quil A. As a further exemplary alternative, the bacteria can be encapsulated within microparticles, such as liposomes. In yet another exemplary alternative, the bacteria can be administered with another immunostimulating macromolecule, such as killed Bordetella, or a tetanus toxoid.

It is preferred that the vaccine of the invention, when in a pharmaceutical preparation, be present in unit dosage forms. The appropriate therapeutically effective dose can be determined readily by those of skill in the art. Accordingly, a pharmaceutical preparation may contain from about 10 about $10^{12}$ of bacteria. More desirably, a pharmaceutical preparation contains about $10^8$ bacteria. It will be understood, however, that the specific dose level for any particular patient will depend upon a variety of factors including the age, general health, sex, and diet of the patient; the time of administration; the route of administration; synergistic effects with any other drugs being administered; and the degree of protection being sought. Of course, the administration can be repeated at suitable intervals if necessary or desirable.

The following examples illustrate the construction of an exemplary vector and transformant of the invention suitable for use as a malaria vaccine. A Salmonella transformant, i.e. an attenuated strain of S. typhimurium, contains a pAS1 plasmid into which is inserted a gene for a CS protein of P. berghei. The E. coli bacteriophage major leftward promoter controls the constitutive expression of the CS gene in the S. typhimurium transformant in the absence of the cl repressor protein. When used to immunize mice, the vaccine induces antigen-specific cell-mediated immune responses and protection from sporozoite challenge in the absence of measurable anti-CS antibodies.


Example 1. Production of An Exemplary Vector pMGB2

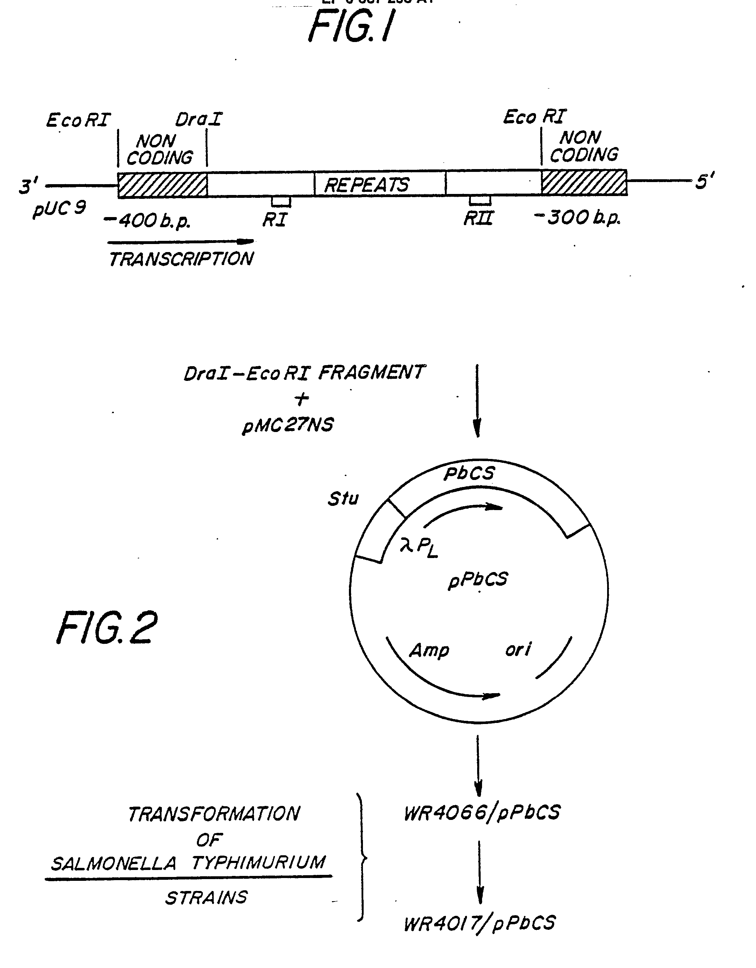

P. berghei DNA from parasitized hamster erythrocytes prepared according to J.E. Weber et al, Exp. Parasitol., 63::295 (1987) was partially digested with DraI and Eco RV. Synthetic EcoRI linkers were ligated to the ends of digested genomic DNA and used to prepare a library in lambda gt10. A clone containing the full length CS gene plus non-coding flanking sequences (shown cross hatched in Fig. 1) was isolated as an Eco RI insert and subcloned into pUC9, a standard E. coli vector commercially available from Bethesda Research Laboratories, Gaithersburg, Md. In Fig. 1, RI and RII refer to conserved regions outside of the repeats as described by Dame et al., Science, supra.

A DraI/Eco RI fragment containing the entire coding region was isolated, and the Eco RI site was filled and blunt end ligated into the StuI site of a lambda $P_L$-based plasmid expression vector pMG27NSTerm. This plasmid is derived from pMG27N- [M. Gross et al., Mol. Cell. Biol., 5:1015 (1985)], and contains a linker inserted adjacent to the methionine initiation codon that contains convenient restriction sites to facilitate insertion of DNA fragments in any of three reading frames and also has downstream termination codons in all three reading frames.

When the resulting construct pMGB2 is transferred into an appropriate E. coli expression host strains, e.g. N5151 [Rosenburg et al, Meth. Enzymol., 101: 122-138 (1983)], a protein is produced that corresponds to the full length P. berghei CS protein with six additional amino acids at its amino terminus (met-asp-pro-trp-arg-lys). The recombinant plasmid was isolated and purified according to procedures described in F. Casse et al., J. Gen. Microbiol, 113:229 (1979).


Example 2. Preparation of a transformed Salmonella strain

pMGB2 was transformed into S. typhimurium WR4066 by a modification described in J. Ou, et al. Recent Advances in Chemotherapy (Ed. Joji Ishigami, University of Tokyo Press, 1985) of the standard methods of S. Cohen et al., Proc Natl. Acad. Sci. U.S.A., 69:2110 (1972) and E. N. Lederberg et al, J. Bacteriol., 119:1072 (1974).

Transformants were selected for ampicillin resistance, colony lifted to nitrocellulose paper, reacted with a monoclonal antibody ("Mab") 3.28.1 directed against P. berghei CS repeat epitopes [G. Furness and D. Rowly, J. Gen Microbiol., 15:140 (1956)] and developed with alkaline phophatase-labeled secondary antibody according to the procedures of H. Sidbery et al, J. Immunol. Meth., 76: 299 (1985). Broth cultures were prepared from a selected colony giving a strong color reaction and plasmid was isolated and transformed into S. typhimurium, strain WR4017. A single colony, WR4017/pMGB2, giving a strong reaction with Mab 3.28.1 was selected to prepare cultures for immunization studies.

Example 3. Cell-Mediated Immunity After Vaccination

Protective cell-mediated immunity was induced by administration of attenuated S. typhimurium, strain WR4017, transformed with the plasmid of Example 1, expressing the P. berghei CS protein. Six to eight week old female BALB/c mice were immunized at week 0 with $10^4$. bacteria in a single subcutaneous dose ("sc") or with $10^9$ or $10^{10}$ bacteria in a single oral dose ("po"). Cultures of liver homogenates from mice receiving WR4017/pMGB2 or untransformed WR4017 by either route were positive when sampled up to 3 weeks after immunization. In the experiment reported in Table I, mice immunized with WR4017 alone and mice immunized with the WR4017/pMGB2 vaccine of the invention were compared after challenge with P. berghei sporozoites. In one experiment (reported at week 9) normal mice and mice immunized with two intravenous ("iv") doses of irradiated sporozoites ("SPZ") were also compared upon challenge. Antibodies to sporozoites were measured by immunofluorescence ("IFA") of air-dried P. berghei sporozoites reacted with serum diluted 1:10 and by an enzyme-linked immunosorbent assay ("ELISA"). The ELISA and IFA assays were performed on coded samples as described in J. F. Young et al., Science, 228: 958 (1985) with the following modifications: (i) optical density for sera diluted 1:100 in ELISA used a 24 amino acid synthetic peptide (DPAPPNAN)₃ conjugated to boiled casein as the capture antigen; (ii) air-dried P. berghei sporozoites were used for IFA. Mice were challenged intravenously with 1500 P. berghei salivary gland sporozoites, and blood films were examined for parasites daily from days 6-12.

Efficacy results in percentages are shown in Table 1 below; ND means not done. Efficacy was calculated as 100 x [1-(% WR4017/pMGB2 infected/% WR4017 infected)]. Mice immunized by either route were protected when challenged with sporozoites 4 to 9 weeks after immunization. Twenty-six of 57 (46%) mice immunized with WR4017/pMGB2 became infected, compared with 32/35 (91%) mice immunized with WR4017 ($X^2$ = 17.62, p = 0.0001). Sixteen of 26 WR4017/pMGB2-immunized mice that did become infected had at least a 1 day delay in onset of parasitemia compared to 29/32 WR4017 control mice that became parasitemic on day 6 (p < 0.001, Student's t-test). Orally administered vaccine was more efficacious than subcutaneous immunization (65% versus 37%, $X^2$ = 4.97, p = 0.026). Mice immunized subcutaneously with WR4017/pMGB2 developed low levels of anti-CS antibodies that were detected at week 4 but not at week 5. Mice immunized orally with larger doses of WR4017/pMGB2 failed to make detectable anti-sporozoite antibodies.

TABLE I

| Time | Immunogen | Route | Dose | IFA | ELISA | # infected/# challenged | Efficacy (%) |
|------|-----------|-------|------|-----|-------|------------------------|--------------|
| Wk 4 | WR4017 | sc | $10^4$ | 0 | .034 | 5/5 | - |
|  | WR4017/pMGB2 | sc | $10^4$ | 1 + | .147 | 5/9 | 44 |
| Wk 5 | WR4017 | sc | $10^4$ | 0 | .057 | 10/10 | - |
|  | WR4017/pMGB2 | sc | $10^4$ | 0 | .073 | 12/18 | 33 |
|  | WR4017 | po | $10^{10}$ | 0 | .060 | 5/5 | - |
|  | WR4017/pMGB2 | po | $10^{10}$ | 0 | .059 | 4/10 | 60 |
|  | WR4017 | po | $10^9$ | 0 | .063 | 5/5 | - |
|  | WR4017/pMGB2 | po | $10^9$ | 0 | 058 | 3/10 | 70 |
| Wk 9 | WR4017 | po | $10^9$ | ND | ND | 7/10 | - |
|  | WR4017/pMGB2 | po | $10^9$ | ND | ND | 2/10 | 71 |
|  | None | - | - | 0 | 0.035 | 7/8 | - |
|  | -SPZ | iv | 75,000 + 20,000 | 4 + | 1.477 | 0/5 | 100 |

Example 4. Delayed type Hypersensitivity

Delayed type hypersensitivity ("DTH") responses to P. berghei sporozoites were shown in mice immunized with (a) radiation-attenuated P. berghei sporozoites ( -SPZ) or (b) the vaccine of Example 2 (WR4017/pMGB2). Mice were immunized with a single oral dose of $10^9$ bacteria, or with two doses (75,000 and 20,000) of irradiated sporozoites. DTH responses were determined 8 or 9 weeks later in groups of 10 mice by measuring hind footpad swelling 24 hr. after injecting phosphate buffered saline ("PBS") or a lysate from 30,000 freeze-thawed sporozoites ("SPZ").

Mice were randomized to receive sporozoite lysate or PBS in the right or left hind footpad. Sporozoite lysate was prepared from freshly dissected salivary gland sporozoites that were pelleted (10,000 X g for 3 minutes), resuspended in PBS to 30,000 sporozoites/25 ul, and subjected to three freeze-thaw cycles (dry ice-ethanol followed by $60^\circ$ C $H_2O$). Sporozoite lysate or PBS (25 ul) was injected with a Hamilton syringe repeating dispenser attached to a 27 ga. needle. Before and 24 hr after injection, footpad thickness was determined to within 0.10 mm using Schnelltaster calipers by an observer who was blinded to both the immunization group and the skin test antigen.

Results are expressed in Table II below as the mean ± standard error ("S.E.") increase in footpad thickness (mm). For statistical analysis, p values were calculated within each group (PBS vs SPZ) and between groups (SPZ reactions in WR4017 controls vs. mice immunized with WR4017/pMGB2 or -SPZ) by Student's one tailed t-test. [NS means not significant.]

TABLE II

| Group | | Mean ± S.E. | | p values | |
|---|---|---|---|---|---|
| | | PBS | SPZ | Within | Between |
| Exp. A | | | | | |
| | WR4017/pMGB2 | 0.00±0.00 | 0.11±0.034 | 0.003 | 0.003 |
| | WR4017 | 0.01±0.001 | 0.00±0.00 | NS | 0.003 |
| | -SPZ | 0.00±0.00 | 0.12±0.036 | 0.003 | |
| Exp. B | | | | | |
| | WR4017/pMGB2 | 0.00±0.00 | 0.06±0.020 | 0.003 | 0.007 |
| | WR4017 | 0.01±0.001 | 0.01±0.001 | NS | 0.03 |
| | -SPZ | 0.00±0.00 | 0.08±0.039 | 0.02 | 0.02 |
| | Normal | 0.00±0.00 | 0.00±0.00 | NS | |

Mice immunized with irradiated P. berghei sporozoites developed DTH reactions in response to inoculation with a sporozoite lysate. Mice immunized orally with WR4017/pMGB2 also developed DTH reactions in response to sporozoite lysates, indicating that the vaccine induced a CS antigen-specific cell-mediated immune response.

Example 5. Combination Immunization Protocol

In experiments carried out substantially as described above, significantly fewer mice were protected by immunization with WR4017 transformed with a PL vector encoding approximately 2/3 of the P. berghei CS protein (lacking C-terminal sequences including a portion of the repeat region) then were protected by a transformant expressing a full length CS protein. Also, when mice were immunized with WR4017 transformed with a PL vector encoding the full length CS protein and then were passively immunized with anti-CS protein monoclonal antibodies, greater than 80% of the mice were protected. This compares to about 40 to 45% of mice treated only with the anti-CS protein monoclonal antibody. These data show that a combination immunization protocol comprising immunization with the vaccine of the invention and internal, i.e., intramuscular or subcutaneous, administration with an antibody-inducing vaccine, such as a vaccine disclosed in European patent application EP-A-192626, incorporated herein by reference, will provide improved protection against disease caused by infective agents.

The above fully describes the invention and demonstrates the CS protein as a target antigen for protective cell-mediated immunity against sporozoites. It is to be appreciated that the invention is not limited to the embodiments particularly described. Analogous vaccines suitable for oral immunization of humans against other malarial infective agents, such as falciparum or vivax malaria, as well as other infectious agents, e.g. virus, bacterial, parasite, may be constructed according to the teachings of the present invention. All such modifications of the invention come within the scope of the following claims.

## Claims

1. A Salmonella bacterial strain transformed with an expression vector comprising a heterologous gene operatively linked to an E. coli promoter sequence.

2. The transformed strain according to claim 1 being capable of constitutive expression of said heterologous gene.

3. The transformed strain according to claim 1 which is an attenuated strain.

4. The transformed strain according to claim 1 selected from the group consisting of Salmonella typhimurium, Salmonella dublin, Salmonella typhii.

5. The transformed strain according to claim 1 wherein said heterologous gene encodes a bacterial, viral or parasitic protein capable of producing protective immunity against an infective agent.

6. The transformed strain according to claim 1 wherein said heterologous gene is a circumsporozoite protein, or a portion thereof, from a selected Plasmodium species.

7. The transformed strain according to claim 6 wherein said Plasmodium species are selected from the group consisting of P. falciparum, P. vivax, P. berghei, P. malariae, P. ovale.

8. The transformed strain according to claim 7 wherein said Plasmodium gene is a full length circumsporozoite protein.

9. The transformed strain according to claim 1 wherein said E. coli promoter sequence is a bacteriophage lambda promoter.

10. The transformed strain according to claim 9 wherein said bacteriophage lambda promoter is a $P_L$ promoter.

11. The transformed strain according to claim 10 wherein the $P_L$ promoter is in a vector having the regulatory region of pAS1.

12. A vaccine capable of inducing cell-mediated immunity against infection in an animal or human by an infective agent comprising an attenuated Salmonella bacterial strain transformed with a vector containing the coding sequence of all, or a portion of, a gene from said infective agent capable of inducing protective immunity, said gene being under control of an E. coli promoter sequence and said transformant being capable of constitutive expression of said heterologous gene in vivo.

13. A vaccine capable of eliciting cell-mediated immunity in an animal or human against malarial infection comprising an attenuated Salmonella bacterial strain transformed with a vector containing a coding sequence for all, or a portion of, a circumsporozoite protein of a selected Plasmodium species under control of a bacteriophage lambda $P_L$ promoter sequence, said transformant capable of constitutive expression of said CS gene.

14. The vaccine according to claim 13 or 14 which is capable of oral administration to a subject.

15. A vaccine as described in any one of claims 13 to 15 for use in therapy.

16. A transformed bacterial strain as described in any one of claims 1 to 11, for use in therapy.

17. A method for expressing a selected heterologous gene comprising culturing in a suitable medium a Salmonella bacterial strain transformed with a vector containing the coding sequence for said heterologous gene under the control of an E. coli promoter sequence.

Claims for the following Contracting States: ES and GR

1. A method for expressing a selected heterologous gene comprising culturing in a suitable medium a Salmonella bacterial strain transformed with a vector containing the coding sequence for said heterologous gene under the control of an E. coli promoter sequence.

2. The method according to claim 1 in which the transformed strain is selected from the group consisting of Salmonella typhimurium, Salmonella dublin, Salmonella typhii.

3. The method according to claim 1 in which said heterologous gene encodes a bacterial, viral or parasitic protein capable of producing protective immunity against an infective agent.

4. The method according to claim 1 in which said heterologous gene is a circumsporozoite protein, or a portion thereof, from a selected Plasmodium species.

5. The method according to claim 4 in which said Plasmodium species is selected from the group consisting of P. falciparum, P. vivax, P. berghei, P. malariae, P. ovale.

6. The method according to claim 5 in which said Plasmodium gene is a full length circumsporozoite protein.

7. The method according to claim 1 wherein said E. coli promoter sequence is a bacteriophage lambda promoter.

8. The method according to claim 7 wherein said bacteriophage lambda promoter is a $P_L$ promoter.

9. The method according to claim 8 wherein the $P_L$ promoter is in a vector having the regulatory region of pAS1.

10. A process for preparing a vaccine capable of inducing cell-mediated immunity against infection in an animal or human by an infective agent comprising bringing into association an attenuated Salmonella bacterial strain transformed with a vector containing the coding sequence of all, or a portion of, a gene from said infective agent capable of inducing protective immunity, said gene being under control of an E. coli promoter sequence and said transformant being capable of constitutive expression of said heterologous gene in vivo, and an acceptable carrier, and, optionally, another vaccinal agent.

11. A process for preparing a vaccine capable of eliciting cell-mediated immunity in an animal or human against malarial infection comprising bringing into association an attenuated Salmonella bacterial strain transformed with a vector containing a coding sequence for all, or a portion of, a circumsporozoite protein of a selected Plasmodium species under control of a bacteriophage lambda $P_L$ promoter sequence, said transformant capable of constitutive expression of said CS gene, and an acceptable carrier.

12. The process according to claim 10 or 11 in which the vaccine prepared is capable of oral administration to the subject.

## FIG.1

EcoRI      DraI                  Eco RI

NON CODING                NON CODING

3'           REPEATS          5'

pUC 9     −400 b.p.     RI        RII     −300 b.p.

TRANSCRIPTION

## FIG.2

DraI−EcoRI FRAGMENT
+
pMC27NS

Stu    PbCS

λP$_L$    pPbCS

Amp    ori

TRANSFORMATION
OF
SALMONELLA TYPHIMURIUM
STRAINS

WR4066/pPbCS

WR4017/pPbCS

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | SCIENCE, vol. 240, 15th April 1988, pages 336-338; J.C. SADOFF et al.: "Oral salmonella typhimurium vaccine expressing circumsporozoite protein protects against malaria" * Whole article * --- | 1-17 | A 61 K 39/112 C 12 N 15/00 A 61 K 39/015// (C 12 N 15/00 C 12 R 1:42 ) (C 12 N 15/00 C 12 R 1:90 ) |
| X | EP-A-0 249 449 (ENTEROVAY RESEARCH PTY. LTD) * Pages 5,6; claims * --- | 1-5,12, 16,17 | |
| X | EP-A-0 250 614 (TIMMIS) * Whole document * --- | 1-5,12, 16,17 | |
| X | EP-A-0 127 153 (THE WELLCOME FOUNDATION) * Whole document * --- | 1-5,12, 16,17 | |
| X | BACTERIAL VACCINES AND LOCAL IMMUNITY, no. 1-2, 17th-19th November 1986, pages 133-141; D. MASKELL et al.: "Attenuated salmonellae as vaccines and carriers of foreign antigens" * Whole article, especially page 134 "Material and Methods" * --- | 1-5,9-12,16, 17 | TECHNICAL FIELDS SEARCHED (Int. Cl.5) C 12 N A 61 K |
| Y | IDEM --- | 6,13-15 | |
| X | VACCINE, vol. 6, no. 2, April 1988, pages 155-160, Butterworth & Co., Guildford, Surrey, GB; R. CURTISS III et al.: "Avirulent salmonella typhimurium Dcya Dcrp oral vaccine strains expressing a streptococcal colonization and virulence antigen" * Whole article * --- -/- | 1-5,12, 16,17 | |

The present search report has been drawn up for all claims

| Place of search | Date of completion of the search | Examiner |
|---|---|---|
| THE HAGUE | 10-10-1989 | SKELLY J.M. |

EPO FORM 1503 03.82 (P0401)

European Patent Office

**EUROPEAN SEARCH REPORT**

Application Number

EP 89 30 7406

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 706 590 (BROENTERPRISES PTY.)<br>* Whole document *<br>--- | 6,13-15 | |
| X | WO-A-8 300 437 (FORMAL)<br>* Page 14-17; claims *<br>--- | 1-5,12,<br>16,17 | |
| X | WO-A-8 503 521 (ENTEROVAX RESEARCH PTY.)<br>* Whole document *<br>--- | 1-5,12,<br>16,17 | |
| X | BIO/TECHNOLOGY, vol. 6, June 1988, page 693; K. NAKAYAMA et al.: "Construction of an asd+ expression-cloning vector: stable maintenance and high level expression of cloned genes in a salmonella vaccine strain"<br>* Page 694, colonne 1, line 17 - colonne 2, line 29 *<br>----- | 1-5,12,<br>16,17 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 10-10-1989 | SKELLY J.M. |